# FASCICULE DE BREVET EUROPEEN

(11) **EP 0 792 637 B1**
(45) Date de publication et mention de la délivrance du brevet: **06.05.1998**
(21) Numéro de dépôt: 97400176.0
(22) Date de dépôt: 27.01.1997
(51) Int. Cl.: A61K 7/48, A61K 7/027, C08L 23/20, C08K 5/10

(54) **Composition cosmétique à appliquer notamment sur les lèvres et utilisations**
Kosmetische Zusammensetzung für Lippen und ihrer Verwendung
Cosmetic composition for lips and use thereof

(30) Priorité: 01.03.1996 FR 9602626
(43) Date de publication de la demande: 03.09.1997
(73) Titulaire: L'OREAL, 75008 Paris (FR)
(72) Inventeur: Arnaud, Pascal, 94000 Creteil (FR)
(74) Mandataire: Dodin, Catherine

(56) Documents cités:
- WO-A-93/19074
- DE-A- 2 360 306
- US-A- 5 085 855

## Description

La présente invention a trait à une composition notamment cosmétique, susceptible d'être appliquée sur la peau et/ou sur les muqueuses, et en particulier sur les lèvres du visage.

Les compositions de rouges à lèvres comprennent généralement des corps gras tels que des huiles et des cires, et des pigments. Elles peuvent permettre l'obtention d'un film mat ou brillant, dépendant notamment de la nature et/ou des proportions des différents constituants.
Ainsi, on sait qu'une bonne dispersion des pigments et/ou des charges, dans la composition, permet d'améliorer la brillance du film.
On sait également qu'il est possible d'améliorer la qualité de la brillance d'un film de rouge à lèvres, par exemple, en augmentant, dans la composition, la proportion de phase grasse, notamment huileuse, par rapport à la phase pigmentaire. D'autre part, certains composés huileux permettent d'obtenir les caractéristiques de brillance adéquates; ceci est le cas, notamment, des composés susceptibles de réfléchir la lumière et ayant un indice de réfraction élevé, tels que certains corps gras huileux. Parmi ceux-ci, on préfère ceux ayant un faible pouvoir de pénétration ou d'absorption dans la peau, tels que les polymères huileux.
Ces composés sont particulièrement intéressants car ils permettent également d'améliorer la rémanence et la tenue du film. En effet, il est connu que la brillance d'un film avait tendance à diminuer au cours du temps, notamment à cause de l'éventuelle mauvaise tenue du film sur le support, de son usure ou de sa migration dans les ridules de la peau, ou encore de son transfert sur d'autres supports.

Toutefois on a constaté que la présence, dans la composition, d'un fort pourcentage de ces polymères huileux, entraîne une diminution des propriétés cosmétiques de ladite composition. On constate ainsi une diminution du caractère glissant de la composition lors de l'application, d'où un étalement sur la peau qui peut sembler malaisé. On constate également, après application, une augmentation du caractère collant de la composition, d'où un inconfort certain sur les lèvres ainsi qu'une qualité esthétique inadéquate.

La présente invention a pour but de proposer une composition qui permette l'obtention d'un film ayant une bonne brillance après application sur le support, tout en conservant des propriétés cosmétiques adéquates.

Un objet de la présente invention est une composition comprenant, dans une phase huileuse, l'association d'au moins un ester aromatique avec au moins un polymère comprenant un motif -[C(CH₃)₂-CH₂]-, ladite phase huileuse ayant un indice de réfraction compris entre 1,4750 et 1,5050.
Un autre objet est une composition comprenant l'association d'au moins un ester aromatique avec au moins un polymère comprenant un motif -[C(CH₃)₂-CH₂]-, et permettant l'obtention d'un film ayant une brillance comprise entre 200 et 600.

Un autre objet de l'invention est l'utilisation de l'association d'au moins un ester aromatique avec au moins un polymère comprenant un motif -[C(CH₃)₂-CH₂]-dans ou pour obtenir une composition permettant l'obtention d'un film brillant. Encore un objet de l'invention est l'utilisation de l'association d'au moins un ester aromatique avec au moins un polymère comprenant un motif -[C(CH₃)₂-CH₂]-dans ou pour obtenir une composition comprenant une phase huileuse ayant un indice de réfraction compris entre 1,4750 et 1,5050.

Par 'film brillant', on entend dans la présente description, un film présentant une mesure de brillance de l'ordre de 200 à 600, de préférence entre 300 et 500. La méthode de mesure de la brillance est décrite avant les exemples.

On a donc constaté que le fait d'incorporer des polymères comprenant un motif -[C(CH₃)₂-CH₂]- en association avec des esters aromatiques permettait une composition qui présente notamment, de bonnes propriétés cosmétiques, telles qu'un glissant, un étalement, une tenue et un collant adéquats, par rapport aux compositions de l'art antérieur qui comprennent uniquement des polymères huileux.

D'autre part, on a constaté que les esters particuliers sélectionnés dans le cadre de la présente invention, à savoir les esters aromatiques, permettent encore d'améliorer la brillance du film, de par le fait qu'ils possèdent également, pour la plupart, un indice de réfraction élevé.

La composition selon l'invention comprend donc au moins un ester aromatique et au moins un polymère ayant un motif -[C(CH₃)₂-CH₂]-, qui peuvent représente la totalité ou une partie seulement de la phase huileuse de la composition, ladite phase huileuse devant par ailleurs avoir un indice de réfraction compris entre 1,4750 et 1,5050.

Ainsi que cela est connu selon l'état de la technique, le changement de direction d'un rayon lumineux passant d'un milieu à un autre est lié aux indices de réfraction de chacun des milieux.
L'indice de réfraction de la phase huileuse selon l'invention est compris entre 1,4750 et 1,5050, mesuré à température ambiante (20-25°C), par exemple à l'aide d'un réfractomètre. De préférence, il est compris entre 1,4800 et 1,5000.

Parmi les esters aromatiques susceptibles d'être utilisés dans le cadre de la présente invention, on peut citer, seul ou en mélange, les monoesters et les polyesters d'acides possédant un groupement aromatique, avec des alcools linéaires ou ramifiés, saturés ou insaturés, ayant 8-30 atomes de carbone. On peut aussi employer des esters d'acides gras, par exemple ayant 8-30 atomes de carbone, avec des alcools ayant un groupement aromatique.
On peut citer, en particulier, les monoesters de l'acide benzoïque et les polyesters de l'acide trimellitique avec des alcools linéaires ou ramifiés, saturés ou insaturés, ayant 8-24 atomes de carbone.
Notamment, on peut citer les benzoates d'alkyle en 12-15 atomes de carbone, les benzoates d'isostéaryle ou d'octyldodécyle, ou encore le laurate de benzyle.
Les esters aromatiques selon l'invention se présentent de préférence sous forme de liquide huileux, ou éventuellement sous forme pâteuse, à température ambiante.

Les polymères ayant un motif -[C(CH₃)₂-CH₂]- selon l'invention sont connus sous le nom CTFA de polybutène ou polyisobutène.
Ils ont de préférence un poids moléculaire moyen de 500-50000, et plus particulièrement compris entre 800 et 10000.
Ils peuvent ainsi se présenter sous forme de liquide ou sous forme pâteuse. Dans le cadre de l'invention, on peut utiliser un polymère unique ou un mélange de ces polymères.
Parmi les polymères commerciaux entrant dans le cadre de l'invention, on peut citer l'INDOPOL H100, l'INDOPOL H300 et l'INDOPOL H1500, de la société AMOCO, ainsi que le PERMETHYL 104A de la société PRESPERSE Inc.

La nature et/ou les quantités d'esters aromatiques et/ou de polymères à motif -[C(CH₃)₂-CH₂]- peut être choisie par l'homme du métier, sur base de ses connaissances générales, de manière à obtenir un mélange (ester + polymère) dont l'indice de réfraction est mesurable. En particulier, ledit mélange se présente de préférence sous la forme d'un mélange liquide et limpide, par exemple ayant une viscosité dynamique à 25°C de 3-6 Pa.s, mesurée avec un viscosimètre rotatif CONTRAVES TV équipé d'un mobile "MS-r4" à la fréquence de 60 Hz, et un indice de réfraction compris entre 1,4750 et 1,5050.

Ainsi, dans un mode particulier de réalisation de l'invention, le mélange (ester + polymère) constitue la totalité de la phase huileuse de la composition, voire la totalité de la phase grasse de la composition.
La phase huileuse de la composition selon l'invention peut comprendre 5-95% en poids d'esters aromatiques, de préférence 8-90% en poids, par rapport au poids total de ladite phase huileuse.
La phase huileuse peut également comprendre 5-95% en poids de polymères à motif -[C(CH₃)₂-CH₂]-, de préférence 10-50% en poids, par rapport au poids total de ladite phase huileuse.

La composition peut se présenter sous forme d'une composition cosmétique, pharmaceutique ou hygiénique. Elle trouve principalement une application en tant que composition à appliquer sur la peau ou les muqueuses, et notamment à appliquer sur les lèvres du visage, telle qu'un rouge à lèvres, un soin ou baume à lèvres, ou une composition à appliquer sur un film de rouge à lèvres, notamment afin d'augmenter la brillance dudit film.
Ladite composition peut alors se présenter sous forme d'un stick ou bâton, ou sous forme d'une pâte souple dont on peut mesurer la viscosité. Dans ce dernier cas, ladite viscosité dynamique à 25°C est de préférence comprise entre 3 et 35 Pa.s, mesurée avec un viscosimètre rotatif CONTRAVES TV équipé d'un mobile "MS-r4" à la fréquence de 60 Hz.
La composition peut encore se présenter sous la forme d'une phase liquide, éventuellement épaissie et/ou gélifiée par des épaississants usuels.

La composition selon l'invention peut égalent être utilisée en tant que produit de soin ou de maquillage de la peau, des ongles, des cils et/ou des cheveux, tel qu'un baume, gel ou lotion capillaire, un gel ou lotion de soin pour les ongles, un mascara ou un eye-liner, voire une crème ou gel pour la peau ou le corps.

Selon l'application envisagée, la composition selon l'invention peut comprendre en outre les constituants usuellement employés dans le type d'application envisagée. Toutefois, l'homme du métier veillera à choisir ces constituants complémentaires, et/ou leur quantité, de manière telle que les propriétés avantageuses de la composition selon l'invention ne soient pas, ou substantiellement pas, altérées par l'adjonction envisagée.

Ainsi, la composition peut comprendre, dans sa phase huileuse, des huiles d'origine végétale, minérale, animale ou synthétique, notamment siliconée et/ou fluorée. On emploie, de préférence, des huiles ayant un indice de réfraction élevé, notamment supérieur à 1,45, afin de ne pas altérer la brillance du film.
Parmi les huiles envisageables, on peut citer les huiles volatiles et les huiles non volatiles, qui peuvent être hydrocarbonées, siliconées et/ou fluorées, cycliques ou linéaires, seules ou en mélange.
Par huile volatile, on entend dans la présente description, toute huile susceptible de s'évaporer au contact de la peau. Parmi les huiles siliconées volatiles, on peut citer la cyclotétradiméthylsiloxane, la cyclopentadiméthylsiloxane, la cyclohexa-diméthylsiloxane et le méthylhexyldiméthylsiloxane. Parmi les huiles hydrocarbonées volatiles, on peut citer les isoparaffines.
Parmi les huiles siliconées, on peut citer les polydiméthylsiloxanes (PDMS) et les alkyldiméthicones, les huiles siliconés phénylées telles que les polyphénylméthylsi-loxanes, les silicones modifiées par des groupements aliphatiques et/ou aromatiques, éventuellement fluorés, ou par des groupements fonctionnels tels que des groupements hydroxyles, thiols et/ou amines.

Parmi les huiles non siliconées, on peut citer l'huile de paraffine, de vaseline, le perhydrosqualène, l'huile d'arara, d'amande douce, de calophyllum, de palme, de ricin, de sésame, d'avocat, de jojoba, d'olive ou de germes de céréales; des esters d'acides gras; des alcools; des acétylglycérides; des octanoates, décanoates ou ricinoléates d'alcools ou de polyalcools.

La composition peut en outre comprendre d'autres corps gras tels que des cires, des gommes et/ou des corps gras pâteux d'origine végétale, minérale, animale ou synthétique, notamment siliconée et/ou fluorée.
On peut ainsi utiliser des triglycérides d'acides gras; des glycérides; des huiles hydrogénées concrètes à 25°C; des lanolines; des esters gras concrets à 25°C. Parmi les cires envisageables, seules ou en mélange, on peut citer les cires animales, végétales, minérales et synthétiques, telles que la cire d'abeilles; la cire de Carnauba, de Candellila, d'Ouricury, du Japon, les cires de fibres de lièges ou de canne à sucre; les cires de paraffine, de lignite; les cires microcristallines; les ozokérites; les cires de polyéthylène et les cires obtenues par synthèse de Fis-cher-Tropsch; les cires de silicone; les cires fluorées.

Les constituants de la phase grasse peuvent en particulier être choisis de manière variée par l'homme du métier afin de préparer une composition ayant les propriétés souhaitées, par exemple en consistance ou en texture.

De préférence, la composition comprend 0-90% en poids de constituants gras autres que les esters aromatiques et les polymères à motif -[C(CH₃)₂-CH₂]-.

Selon l'application envisagée, la composition peut comprendre également une phase particulaire, qui peut être présente à raison de 0-25% en poids, de préférence 0-10% en poids, et qui peut comprendre des pigments et/ou des nacres et/ou des charges habituellement utilisés dans les compositions cosmétiques.
La nature et la quantité de pigments et/ou de charges sera de préférence choisie de manière judicieuse par l'homme du métier, afin de ne pas entraîner une diminution trop importante de la brillance du film. Dans cette optique, la quantité de pigments sera de préférence limitée à 10% en poids par rapport au poids total de la composition, et la quantité de charges sera limitée à 5% en poids.
Ainsi, les pigments peuvent être présents dans la composition à raison de 0-15% en poids de la composition finale, de préférence 0-10% en poids. Ils peuvent être blanc ou colorés, minéraux et/ou organiques. On peut citer, parmi les pigments minéraux, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.

Les nacres peuvent être présentes dans la composition à raison de 0-20% en poids, de préférence 0-10% en poids. Parmi les nacres envisageables, on peut citer le mica recouvert d'oxyde de titane, d'oxyde de fer, de pigment naturel ou d'oxychlorure de bismuth ainsi que le micatitane coloré.
Les charges, qui peuvent être présentes à raison de 0-15% en poids, de préférence 0-5% en poids, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, la nacre naturelle, le nitrure de bore, les microsphères telles que l'Expancel (Nobel Industrie), le polytrap (Dow Corning) et les microbilles de résine de silicone (Tospearls de Toshiba, par exemple).

La composition peut comprendre en outre tout additif usuellement utilisé dans le domaine cosmétique, tel que des antioxydants, des parfums, des huiles essentielles, des conservateurs, des actifs cosmétiques, des hydratants, des vitamines, des acides gras essentiels, des sphingolipides, des agents autobronzants tels que la DHA, des filtres solaires, des tensioactifs, des polymères liposolubles notamment hydrocarbonés, tels que les polyalkylènes, les polyacrylates et les polymères siliconés compatibles avec les corps gras.

L'invention est illustrée plus en détail dans les exemples suivants.

### Mesure de la brillance

La mesure de la brillance des compositions selon l'invention est effectuée à l'aide d'un photogoniomètre usuel.
Les compositions sont maintenues à une température de 25°C; on étale sur une plaque de verre à 33°C un film d'une épaisseur de 60 microns.
Le rayon incident arrive sur la plaque avec un angle de -30° par rapport à la normale. La réflexion spéculaire S est mesurée à +30° et la réflexion diffuse D est mesurée à 0°, par rapport à la normale.
La brillance B est déterminée comme étant le rapport S/D.

### Exemple 1

On prépare la composition suivante (% en poids):

| | |
|---|---|
| . polybutène (Indopol H1500 de Amoco) | 45% |
| . benzoate d'alkyle en C12-C15 (FinsolvTN de Finetex) | 33% |
| . copolymère PVP/hexadécène | 22% |

L'indice de réfraction du mélange des trois constituants est de 1,4905.

Les constituants sont chauffés et mélangés à 90°C jusqu'à obtention d'un mélange homogène.

Après refroidissement, on obtient une composition liquide et limpide, qui peut être appliquée sur un film de rouge à lèvres pour en augmenter la brillance

### Exemple 2

On prépare un brillant à lèvres ayant la composition suivante (% en poids) :

| | |
|---|---|
| . polybutène (Indopol H100; poids moléculaire 965) | 45% |
| . trimellitate de tridécyle (DUB TMTD de Stearinerie Dubois) | 20% |
| . cire de polyéthylène | 10% |
| . nacres | 4% |
| . conservateurs, antioxydants | qs |
| . octyldodécanol | qsp 100% |

L'indice de réfraction de la phase huileuse comprenant 'polybutène + trimellitate de tridécyle + octyldodécanol' est de 1,4823.

Les corps gras sont mélangés et chauffés à 90°C jusqu'à obtention d'un mélange homogène. On ajoute les autres constituants puis on coule le mélange dans des moules adéquats.
On obtient ainsi un brillant à lèvres qui permet l'obtention d'un film présentant une bonne brillance, ainsi que des caractéristiques cosmétiques adéquates.

### Exemple 3

On prépare un rouge à lèvres ayant la composition suivante (% en poids) :

| | |
|---|---|
| . polybutène (Indopol H1500; poids moléculaire 2160) | 15% |
| . benzoate d'alkyles en C12-C15 (FinsolvTN de Finetex) | 20% |
| . trimellitate de tridécyle (DUB TMTD de Stearinerie Dubois) | 15% |
| . phényltriméthicone (DC556) | 5% |
| . malate de diisostéaryle | 7% |
| . lanoline | 13% |
| . pigments | 10% |
| . conservateurs, antioxydants | qs |
| . cires (polyéthylène, Candellila, Carnauba) | qsp 100% |

L'indice de réfraction de la phase huileuse, qui comprend 'polybutène + benzoates d'alkyles + trimellitate de tridécyle + phényltriméthicone + malate de diisostéaryle' est de 1,4833.

Les constituants sont mélangés et chauffés à 95°C. Après homogénéisation et broyage, le mélange est coulé à 95°C dans des moules adéquats.
On obtient un stick de rouge à lèvres présentant de bonnes propriétés cosmétiques et permettant l'obtention d'un film brillant sur les lèvres.

## Revendications

1. Composition comprenant, dans une phase huileuse, l'association d'au moins un ester aromatique avec au moins un polymère comprenant un motif -[C(CH₃)₂-CH₂]-, ladite phase huileuse ayant un indice de réfraction compris entre 1,4750 et 1,5050.

2. Composition selon la revendication 1, dans laquelle l'indice de réfraction de la phase huileuse est compris entre 1,4800 et 1,5000.

3. Composition comprenant l'association d'au moins un ester aromatique avec au moins un polymère comprenant un motif -[C(CH₃)₂-CH₂]-, et permettant l'obtention d'un film ayant une brillance comprise entre 200 et 600.

4. Composition selon la revendication 3, permettant l'obtention d'un film ayant une brillance comprise entre 300 et 500.

5. Composition selon l'une des revendications précédentes, dans laquelle l'ester aromatique est choisi parmi, seul ou en mélange, les monoesters et les polyesters d'acides possédant un groupement aromatique, avec des alcools linéaires ou ramifiés, saturés ou insaturés, ayant 8-30 atomes de carbone; les esters d'acides gras ayant 8-30 atomes de carbone, avec des alcools ayant un groupement aromatique.

6. Composition selon l'une des revendications précédentes, dans laquelle l'ester aromatique est choisi parmi, seul ou en mélange, les monoesters de l'acide benzoïque et les polyesters de l'acide trimellitique avec des alcools linéaires ou ramifiés, saturés ou insaturés, ayant 8-24 atomes de carbone.

7. Composition selon l'une des revendications précédentes, dans laquelle l'ester aromatique est choisi parmi, seul ou en mélange, les benzoates d'alkyle en 12-15 atomes de carbone, les benzoates d'isostéaryle ou d'octyldodécyle, et le laurate de benzyle.

8. Composition selon l'une des revendications précédentes, dans laquelle les polymères ayant un motif -[C(CH₃)₂-CH₂]- ont un poids moléculaire moyen de 500-50000, de préférence de 800-10000.

9. Composition selon l'une des revendications précédentes, dans laquelle le mélange (ester + polymère) présente une viscosité dynamique à 25°C de 3-6 Pa.s.

10. Composition selon l'une des revendications précédentes, dans laquelle la phase huileuse comprend 5-95% en poids d'esters aromatiques, de préférence 8-90% en poids, par rapport au poids total de ladite phase huileuse.

11. Composition selon l'une des revendications précédentes, dans laquelle la phase huileuse comprend 5-95% en poids de polymères à motif -[C(CH₃)₂-CH₂]- de préférence 10-50% en poids, par rapport au poids total de ladite phase huileuse.

12. Composition selon l'une des revendications précédentes, dans laquelle la phase huileuse comprend en outre des huiles d'origine végétale, minérale, animale ou synthétique, notamment siliconée et/ou fluorée, ayant un indice de réfraction supérieur à 1,45.

13. Composition selon l'une des revendications 1-9, dans laquelle l'association d'au moins un ester aromatique avec au moins un polymère comprenant un motif -[C(CH₃)₂-CH₂]- représente la totalité de la phase huileuse de la composition.

14. Composition selon l'une des revendications précédentes, comprenant en outre d'autres corps gras choisis parmi les cires, les gommes et/ou les corps gras pâteux d'origine végétale, minérale, animale ou synthétique, notamment siliconée et/ou fluorée.

15. Composition selon l'une des revendications précédentes, comprenant en outre 0-25% en poids, de préférence 0-10% en poids, d'une phase particulaire.

16. Composition selon l'une des revendications précédentes, dans laquelle la quantité de pigments est inférieure à 10% en poids et/ou la quantité de charges est inférieure à 5% en poids, par rapport au poids total de la composition.

17. Composition selon l'une des revendications précédentes, se présentant sous la forme d'une composition cosmétique, pharmaceutique ou hygiénique.

18. Composition selon l'une des revendications précédentes, se présentant sous la forme d'une composition à appliquer sur les lèvres du visage telle qu'un rouge à lèvres, un soin ou baume à lèvres, ou une composition à appliquer sur un film de rouge à lèvres.

19. Composition selon l'une des revendications 1-17, se présentant sous la forme d'un produit de soin ou de maquillage de la peau, des ongles, des cils et/ou des cheveux, tel qu'un baume, gel ou lotion capillaire, un gel ou lotion de soin pour les ongles, un mascara ou un eye-liner, une crème ou gel pour la peau ou le corps.

20. Utilisation de l'association d'au moins un ester aromatique avec au moins un polymère comprenant un motif -[C(CH₃)₂-CH₂]- dans ou pour obtenir une composition permettant l'obtention d'un film brillant.

21. Utilisation selon la revendication 20, dans laquelle le film possède une brillance comprise entre 200 et 600, de préférence 300 à 500.

22. Utilisation de l'association d'au moins un ester aromatique avec au moins un polymère comprenant un motif -[C(CH₃)₂-CH₂]- dans ou pour obtenir une composition comprenant une phase huileuse ayant un indice de réfraction compris entre 1,4750 et 1,5050.

23. Utilisation selon l'une des revendications 20 à 22, dans une composition cosmétique, pharmaceutique ou hygiénique.

24. Utilisation selon l'une des revendications 20 à 23, dans un rouge à lèvres, un soin ou baume à lèvres, une composition à appliquer sur un film de rouge à lèvres, un produit de soin ou de maquillage de la peau, des ongles, des cils et/ou des cheveux, tel qu'un baume, gel ou lotion capillaire, un gel ou lotion de soin pour les ongles, un mascara ou un eye-liner, une crème ou gel pour la peau ou le corps.

## Claims

1. Composition comprising, in an oily phase, the combination of at least one aromatic ester with at least one polymer comprising a -[C(CH₃)₂-CH₂]- unit, the said oily phase having a refractive index of between 1.4750 and 1.5050.

2. Composition according to Claim 1, in which the refractive index of the oily phase is between 1.4800 and 1.5000.

3. Composition comprising the combination of at least one aromatic ester with at least one polymer comprising a -[C(CH₃)₂-CH₂]- unit which makes it possible to obtain a film having a gloss of between 200 and 600.

4. Composition according to Claim 3 which makes it possible to obtain a film having a gloss of between 300 and 500.

5. Composition according to one of the preceding claims, in which the aromatic ester is chosen, alone or as a mixture, from monoesters and polyesters of acids possessing an aromatic group with saturated or unsaturated, linear or branched alcohols having 8-30 carbon atoms; or esters of fatty acids having 8-30 carbon atoms with alcohols having an aromatic group.

6. Composition according to one of the preceding claims, in which the aromatic ester is chosen, alone or as a mixture, from monoesters of benzoic acid and polyesters of trimellitic acid with saturated or unsaturated, linear or branched alcohols having 8-24 carbon atoms.

7. Composition according to one of the preceding claims, in which the aromatic ester is chosen, alone or as a mixture, from alkyl benzoates in which the alkyl group contains 12-15 carbon atoms, isostearyl or octyldodecyl benzoates and benzyl laurate.

8. Composition according to one of the preceding claims, in which the polymers having a -[C(CH₃)₂-CH₂]- unit have an average molecular weight of 500-50,000, preferably of 800-10,000.

9. Composition according to one of the preceding claims, in which the (ester + polymer) mixture exhibits a dynamic viscosity at 25°C of 3-6 Pa.s.

10. Composition according to one of the preceding claims, in which the oily phase comprises 5-95% by weight of aromatic esters, preferably 8-90% by weight, with respect to the total weight of the said oily phase.

11. Composition according to one of the preceding claims, in which the oily phase comprises 5-95% by weight of polymers containing a -[C(CH₃)₂-CH₂]- unit, preferably 10-50% by weight, with respect to the total weight of the said oily phase.

12. Composition according to one of the preceding claims, in which the oily phase additionally comprises oils of vegetable, mineral, animal or synthetic origin, in particular silicone and/or fluorinated origin, having a refractive index of greater than 1.45.

13. Composition according to one of Claims 1-9, in which the combination of at least one aromatic ester with at least one polymer comprising a -[C(CH₃)₂-CH₂]- unit represents all of the oily phase of the composition.

14. Composition according to one of the preceding claims, additionally comprising other fatty substances chosen from waxes, gums and/or pasty fatty substances of vegetable, mineral, animal or synthetic origin, in particular silicone and/or fluorinated origin.

15. Composition according to one of the preceding claims, additionally comprising 0-25% by weight, preferably 0-10% by weight, of a particulate phase.

16. Composition according to one of the preceding claims, in which the amount of pigments is less than 10% by weight and/or the amount of fillers is less than 5% by weight, with respect to the total weight of the composition.

17. Composition according to one of the preceding claims, which is provided in the form of a cosmetic, pharmaceutical or hygienic composition.

18. Composition according to one of the preceding claims, which is provided in the form of a composition to be applied on the lips of the face, such as a lipstick or a lip care or balm, or a composition to be applied on a lipstick film.

19. Composition according to one of Claims 1-17, which is provided in the form of a care or make-up product for the skin, nails, eyelashes and/or hair, such as a hair balm, gel or lotion, a gel or lotion for caring for the nails, a mascara or an eyeliner, or a cream or gel for the skin or body.

20. Use of the combination of at least one aromatic ester with at least one polymer comprising a -[C(CH₃)₂-CH₂]- unit in or for obtaining a composition which makes it possible to obtain a glossy film.

21. Use according to Claim 20, in which the film has a gloss of between 200 and 600, preferably 300 to 500.

22. Use of the combination of at least one aromatic ester with at least one polymer comprising a -[C(CH₃)₂-CH₂]- unit in or for obtaining a composition comprising an oily phase having a refractive index of between 1.4750 and 1.5050.

23. Use according to one of Claims 20 to 22 in a cosmetic, pharmaceutical or hygienic composition.

24. Use according to one of Claims 20 to 23 in a lipstick, a lip care or balm, a composition to be applied on a lipstick film, or a care or make-up product for the skin, nails, eyelashes and/or hair, such as a hair balm, gel or lotion, a gel or lotion for caring for the nails, a mascara or an eyeliner, or a cream or gel for the skin or body.

## Patentansprüche

1. Zusammensetzung, die in einer öligen Phase die Kombination mindestens eines aromatischen Esters mit mindestens einem Polymer, das eine Einheit -[C(CH₃)₂-CH₂]- aufweist, enthält, wobei die ölige Phase einen Brechungsindex aufweist, der im Bereich von 1,4750 bis 1,5050 liegt.

2. Zusammensetzung nach Anspruch 1, wobei der Brechungsindex der öligen Phase im Bereich von 1,4800 bis 1,5000 liegt.

3. Zusammensetzung, die die Kombination mindestens eines Esters mit mindestens einem Polymer, das eine Einheit -[C(CH₃)₂-CH₂]- aufweist, enthält, mit der ein Film mit einen Glanz, der im Bereich von 200 bis 600 liegt, erzeugt werden kann.

4. Zusammensetzung nach Anspruch 3, mit der ein Film erzeugt werden kann, der einen Glanz von 300 bis 500 aufweist.

5. Zusammensetzung nach einem der vorhergehenden Ansprüche, wobei der aromatische Ester, allein oder im Gemisch, unter den Monoestern und den Polyestern von Säuren, die eine aromatische Gruppe aufweisen, mit geradkettigen oder verzweigten, gesättigten oder ungesättigten Alkoholen mit 8 bis 30 Kohlenstoffatomen und Estern von Fettsäuren mit 8 bis 30 Kohlenstoffatomen mit Alkoholen, die eine aromatische Gruppe aufweisen, ausgewählt ist.

6. Zusammensetzung nach einem der vorhergehenden Ansprüche, wobei der aromatische Ester, allein oder im Gemisch, unter den Monoestern der Benzoesäure und den Polyestern von Trimellitsäure mit geradkettigen oder verzweigten, gesättigten oder ungesättigten Alkoholen mit 8 bis 24 Kohlenstoffatomen ausgewählt ist.

7. Zusammensetzung nach einem der vorhergehenden Ansprüche, wobei der aromatische Ester, allein oder im Gemisch, unter Alkylbenzoaten mit 12 bis 15 Kohlenstoffatome, Isostearylbenzoat und Octyldodecylbenzoat und Benzyllaurat ausgewählt ist.

8. Zusammensetzung nach einem der vorhergehenden Ansprüche, wobei die Polymere mit einer Einheit -[C(CH₃)₂-CH₂]- ein mittleres Molekulargewicht von 500 bis 50 000 und vorzugsweise 800 bis 10 000 aufweisen.

9. Zusammensetzung nach einem der vorhergehenden Ansprüche, wobei das (Ester + Polymer)-Gemisch bei 25 °C eine dynamische Viskosität von 3 bis 6 Pa·s aufweist.

10. Zusammensetzung nach einem der vorhergehenden Ansprüche, wobei die ölige Phase 5 bis 95 Gew.-% und vorzugsweise 8 bis 90 Gew.-% aromatische Ester, bezogen auf das Gesamtgewicht der öligen Phase, enthält.

11. Zusammensetzung nach einem der vorhergehenden Ansprüche, wobei die ölige Phase 5 bis 95 Gew.-% und vorzugsweise 10 bis 50 Gew.-% Polymere mit Einheiten -[C(CH₃)₂-CH₂]-, bezogen auf das Gesamtgewicht der öligen Phase, enthält.

12. Zusammensetzung nach einem der vorhergehenden Ansprüche, wobei die ölige Phase außerdem Öle pflanzlicher, mineralischer, tierischer oder synthetischer Herkunft, insbesondere Siliconöle und/oder fluorierte Öle, mit einem Brechungsindex von größer als 1,45 enthält.

13. Zusammensetzung nach einem der Ansprüche 1 bis 9, wobei die Kombination mindestens eines aromatischen Esters mit mindestens einem Polymer, das eine Einheit -[C(CH₃)₂-CH₂]- enthält, die gesamte ölige Phase der Zusammensetzung ausmacht.

14. Zusammensetzung nach einem der vorhergehenden Ansprüche, die außerdem weitere Fettsubstanzen enthält, die unter Wachsen, Gummen/Gummis und/oder pastösen Fettsubstanzen pflanzlicher, anorganischer, tierischer oder synthetischer Herkunft, die insbesondere siliconhaltig und/oder fluoriert sind, ausgewählt sind.

15. Zusammensetzung nach einem der vorhergehenden Ansprüche, die außerdem 0 bis 25 Gew.-% und vorzugsweise 0 bis 10 Gew.-% einer Partikel-Phase enthält.

16. Zusammensetzung nach einem der vorhergehenden Ansprüche, wobei die Menge der Pigmente kleiner als 10 Gew.-% und/oder die Menge der Füllstoffe kleiner als 5 Gew.-%, bezogen auf das Gesamtgewicht der Zusammensetzung, ist.

17. Zusammensetzung nach einem der vorhergehenden Ansprüche, die in Form einer kosmetischen oder pharmazeutischen Zusammensetzung oder einer Hygiene-Zusammensetzung vorliegt.

18. Zusammensetzung nach einem der vorhergehenden Ansprüche, die in Form einer Zusammensetzung, die auf die Lippen des Gesichts aufgetragen wird, wie z.B. in Form eines Lippenstifts, eines Lippenpflegemittels oder eines Lippenbalsams, oder in Form einer Zusammensetzung, die auf einen Lippenstiftfilm aufgetragen wird, vorliegt.

19. Zusammensetzung nach einem der Ansprüche 1 bis 17, die in Form eines Pflegeprodukts oder Schminkprodukts für die Haut, die Nägel, die Wimpern und/oder die Haare vorliegt, wie z.B. in Form eines Balsams, Gels oder einer Lotion für die Haare, eines Pflegegels oder einer Pflegelotion für die Nägel, eines Mascaras oder eines Eyeliners, einer Creme oder eines Gels für die Haut oder den Körper.

20. Verwendung der Kombination mindestens eines aromatischen Esters mit mindestens einem Polymer, das eine Einheit -[C(CH₃)₂-CH₂]- aufweist, in einer oder für die Herstellung einer Zusammensetzung, mit der ein glänzender Film erzeugt werden kann.

21. Verwendung nach Anspruch 20, wobei der Film einen Glanz im Bereich von 200 bis 600 und vorzugsweise im Bereich von 300 bis 500 aufweist.

22. Verwendung der Kombination mindestens eines aromatischen Esters mit mindestens einem Polymer, das eine Einheit -[C(CH₃)₂-CH₂]- aufweist, in einer oder für die Herstellung einer Zusammensetzung, die eine ölige Phase enthält, die einen Brechungsindex im Bereich von 1,4750 bis 1,5050 aufweist.

23. Verwendung nach einem der Ansprüche 20 bis 22 in einer kosmetischen oder pharmazeutischen Zusammensetzung oder einer Hygiene-Zusammensetzung.

24. Verwendung nach einem der Ansprüche 20 bis 23 in einem Lippenstift, einem Lippenpflegemittel oder einem Lippenbalsam, einer Zusammensetzung, die auf einen Lippenstiftfilm aufgetragen wird, einem Pflegeprodukt oder Schminkprodukt für die Haut, die Nägel, die Wimpern und/oder die Haare, wie z.B. einem Balsam, Gel oder einer Lotion für die Haare, einem Pflegegel oder einer Pflegelotion für die Nägel, einer Mascara oder einem Eyeliner, einer Creme oder einem Gel für die Haut oder den Körper.
